# EUROPEAN PATENT SPECIFICATION

(11) **EP 4 450 039 B1**
(45) Date of publication and mention of the grant of the patent: **21.10.2026**
(21) Application number: 24168295.4
(22) Date of filing: 03.04.2024
(51) Int. Cl.: A61F 13/511, A61F 13/512, A61F 13/84

(54) **SOLID SUPPORTS COATED WITH OR INCORPORATING PROBIOTICS AND METABOLITES OF PROBIOTIC BACTERIA**
FESTE TRÄGER, DIE MIT PROBIOTIKA UND METABOLITEN PROBIOTISCHER BAKTERIEN BESCHICHTET SIND ODER DIESE ENTHALTEN
SUPPORTS SOLIDES ENDUITS DE PROBIOTIQUES ET DE MÉTABOLITES DE BACTÉRIES PROBIOTIQUES OU LES INCORPORANT

(30) Priority: 17.04.2023 IT 202300007371
(43) Date of publication of application: 23.10.2024
(73) Proprietor: Texol S.R.L., 65020 Alanno (PE) (IT)
(72) Inventor: Odoardi, Renzo Marcello, 65027 Scafati PE (IT); Turacchio, Sabrina, 65020 Alanno PE (IT); Gagliardini, Alessandro, 65020 Alanno PE (IT); Cimini, Carmine, 65020 Alanno PE (IT)
(74) Representative: Montemurro, Riccardo

(56) References cited:
- EP-A1- 1 263 483
- EP-B1- 1 263 483
- US-A1- 2003 012 810

## Description

### FIELD OF THE INVENTION

The invention discloses materials and process for their manufacturing, that can be incorporated in products thought for being in contact with human body such as absorbent hygienic products (baby diapers, adult incontinence diapers and women's personal hygiene products) comprising spores, soluble metabolites and/or cell lysates of probiotic micro-organism.

Materials and products coated with or incorporating such spores, soluble metabolites and/or cell lysates of probiotic micro-organism will deliver beneficial effects to human when in contact with the body.

### BACKGROUND OF THE INVENTION

The use of conventional pad/panty liners can increase temperature, skin surface moisture, pH, thereby significantly changing the microclimate of the vulva which allows undesirables microorganisms to overgrow and causing odors, itching, intimate discomforts.

Overall, during menstruation, the level of probiotic lactobacilli decline and changes in normal vaginal flora causes change in pH which allows a variety of anaerobes to overgrow and give vaginal imbalance which may result in fish odor, discharge, itching, vaginal rash, infections.

For example, bacterial vaginosis, caused by Candida albicans, Gardnerella vaginalis and Trichomonas spp, is an ecological imbalance of the vaginal microbiome with a decrease in vaginal acidity and concentration of lactobacilli accompanied by an increase (100 fold more) in the concentration of other microorganisms.

Also, urinary tract infections are caused by a variety of bacteria including E. Coli found in feces. Because bowel, vagina and urethra are very close it is easy for the bacteria to spread to the urethra. A health vaginal flora helps to prevent harmful bacteria from entering in the urinary tract.

The unfavorable environment under the diaper due to the prolonged occlusion and exposure to excreta damages the skin integrity, changes skin pH, gives overhydration and changes in skin microbial flora and producing diaper dermatitis. A recognized factor in diaper dermatitis is the change of the skin microbiome with associated pathogenic strains Candida albicans and Staphylococcus aureus.

### Probiotics

Probiotics are friendly bacteria which confer health benefits to the humans.

Application on skin of probiotics may have the same benefits as oral probiotics in preserving/restoring the microbic ecological balance that promotes human health and beauty.

Probiotics are not pathogenic for humans, likewise their presence creates a hostile environment for other pathogenic microorganisms to survive.

Probiotics may be beneficial in defending vulvar ecosystem, promoting heathy vaginal microbiome, thus preventing vaginal rash, itching, intimate discomforts and malodors, intimate infections and in baby and adult diapers to prevent diaper rash.

Soluble metabolites, cell lysates and ferment extracts are obtained by fermentation of probiotic bacteria. They are a blend of dead bacteria, cell wall fragments and soluble metabolites (organic acids, free fatty acids, hydrogen peroxide, bacteriocins).

The term "soluble metabolite" refers to a metabolite or metabolites present in the supernatant of a probiotic cell culture from which the cells have been removed, typically it is composed by a blend of organic acids, free fatty acids, hydrogen peroxide and bacteriocins.

In particular the bacteriocins (Lantibiotics, Non-Lantibiotics, Plantaricins, Lactacines, Lactoccines, Lactococcin, Nisin, Leucocin) are peptides or proteins with antimicrobial activity.

Nowadays, bacteriocins have been utilized for food preservation, among them Nisine is the most popular and widely used as natural food packaging preservative.

The term "cell lysate" or "lysate" refers to probiotic cells which have been lysed by any suitable means. For example, the cells may be lysed by sonication, homogenization, shearing or chemical lysis.

These semi-active, non-replicating probiotics retain activities comparable to the live forms, likewise the living probiotics are capable of interfering with the growth of other undesirable microorganisms and can elicit certain immune responses on the skin improving skin barrier functions and vaginal health.

Cosmetic compositions for local application containing probiotics and probiotic metabolites provide skin benefits (hydration, anti-inflammatory, antimicrobial, improving atopic eczema, atopic dermatitis, healing of burn and scars, skin-rejuvenating properties) and could be appropriate to prevent problems related to skin sensitivity by improving skin resistance to external stress.

Probiotics and/or their soluble metabolite and/or a cell lysate are used in compositions for topical treatment of sensitive skin, prevent and/or treat sensations of discomfort and disorders associated with skin barrier functions (WO2012150269; ES2751994T3; WO2020165919A1).

Probiotics are also incorporated into personal hygiene products such as sanitary napkins panty liners, tampons, baby and adult diapers.

For example patent WO2010/128906 relates to sanitary articles containing a combination of lactic acid producing probiotic bacteria with oxidized lipids; in US 2004/0243076 is described the use of probiotic bacteria in dried form in combination with adsorbent powder, in WO99/17813 and US2003/001281 is described the use of spores of probiotics in absorbent articles.

EP1263483A1 and US2003/012810 A1 disclose thermoplastic perforated films used as components in sanitary articles, as well as treatments with spores and metabolites for anti-micotic and anti-microbic effect.

In these inventions probiotics are applied inside the structure of the product, dispersed in the fibers of the absorbent core, in a way that do not permit microorganisms to go in direct contact with body and skin, therefore not going in direct contact with the human microbiota.

Consequently, the benefits of the presence of probiotics are lost and remain limited only to control odors associated with body fluids absorbed into the structure of the hygienic product.

Another concern is the viability of the microorganisms during process making, storages and shelf life of the products because bacteria rapidly lose viability under moist conditions, it is therefore important that the products are not exposed to moisture.

One way to partly overcome this problem has been to supply products with freeze-dried bacteria. However, if the bacteria in the products are not protected from moisture after manufacturing of the products, the air humidity will subsequently kill the bacteria and the shelf-life of such products will then be shortened.

Incorporating viable micro-organisms in synthetic materials to be able to integrate these beneficial properties is not straightforward.

Indeed, materials such as synthetic fibers, plastic films are prepared under high temperature and/or high-pressure conditions (e.g. extrusion, pelletizing, molding, thermo-forming, compression molding, injection molding, vacuum-forming, foaming, hot-melting) and micro-organisms, to be effectively incorporated therein, have to remain viable during high-pressure and/or high-temperature conditions applied before and during the production process of the material.

These high temperatures (and possibly desiccation), often combined with high-pressure processes, are detrimental of the viability of micro-organisms.

For instance, the processing temperature of polymers is may be above 1000C, which even at normal pressure conditions, is too high for the incorporation of living material.

The implantation of living, active micro-organisms at temperatures of this level is impossible without fatal consequences for these organisms.

Thus, even if the organisms which are introduced would perform useful activity at normal ambient temperatures, if incorporation of living or viable organisms during the production of the material is not achievable, no benefit can be obtained.

### SUMMARY OF THE INVENTION

The present invention relates to materials, in particular thermoplastic perforated films or non-woven fabric materials, and methods for producing them, comprising spores, soluble metabolites and/or cell lysates of probiotic micro-organism. In particular, said spores, soluble metabolites and/or cell lysates of probiotic micro-organism areincorporated into the polymer matrix of the thermoplastic perforated film or incorporated within the fibres of the non-woven fabric material, or or are applied as coating on the surface of the thermoplastic perforated film or of the non-woven fabric material, to create a surface with beneficial properties for the skin, body, and/or intimate areas.

Spores of specific probiotic bacteria proved to be safe, non-pathogenic, may be incorporated on a solid support to be used as top layer in a finished product.

Such compositions and methods will allow the incorporation of viable micro-organisms, wherein the micro-organisms remain viable during the conditions applied for production process, materials and product storage until the product is in use.

Once the product is in use the spores will become active upon direct contact with body and or body fluids and will be capable to directly interact with body/skin microbiota thus being able to deliver beneficial effects of probiotics in preserving/restoring the microbial ecological balance on which human health and beauty depend.

Metabolites and/or cell lysates and ferment extracts of probiotic bacteria incorporated in the material are used as top layer in a finished product for personal hygiene.

Such compositions are thought to guarantee the best beneficial effects thanks the optimal contact with the body during the wearing of the product.

Once the product is in use the metabolites and/or cell lysates of probiotic bacteria, upon direct contact with body and or body fluids, will be capable to directly interact with body/skin microbiota thus being able to deliver their beneficial effects in preserving/restoring the microbial ecological balance on which human health and beauty depend.

Materials containing probiotics or their metabolites may give benefits in feminine pads and pantyliners to defend vulvar ecosystem and promoting a heathy vaginal microbiome and in adult and baby diapers to prevent diaper rash.

Objects of the present invention are:
- a thermoplastic perforated film comprising spores, soluble metabolites and/or cell lysates of probiotic micro-organism;
- a method of preparing a thermoplastic film comprising spore, soluble metabolite and/or cell lysate of probiotic micro-organism into the polymer matrix comprising the following steps:
   i) mixing spores, soluble metabolites and/or cell lysates of probiotic micro-organsim with pellets of a thermoplastic polymer in order to provide a mixture with a total probiotic concentration from 0,01% to 10% w/w;
   ii) extruding said mixture obtained on point i) at a temperature from 100°C to 350°C, a pressure from 0,1 MPa to 40,0 MPa, average residence times from 1 to 10 min; or
   pelletizing, thermos-molding, compression molding, injection molding, thermo forming, impregnating, vacuum-forming, foaming or hot-melting said mixture obtained on point i) in order to obtain a thermoplastic film;
- a method of preparing a thermoplastic film coated with spore, soluble metabolite and/or cell lysate of probiotic micro-organism, comprising the following steps:
   a) mixing spores, soluble metabolites and/or cell lysates of probiotic micro-organism in order to provide a mixture with a total probiotic concentration from 0,01% to 10% w/w with respect to the weight of the thermoplastic film, optionally with additives;
   b) spraying or coating a thermoplastic film with said mixture obtained in step a);
- a method for the preparation of a perforated thermoplastic film coated with spores, soluble metabolites and/or cell lysates of probiotic micro-organism, comprising the following steps:
   a) introducing a thermoplastic film sheet through a perforating nip (5) formed by a drum (4) carrying perforating pins and a female roll (3), while the perforating pins applied on the drum pass through a probiotic materials applicator (6) before becoming in contact with the thermoplastic film sheet;
   b) keeping in contact the perforated thermoplastic film sheet with the pins of the drum roll up to a phase of separation from the pins due to the action of a vacuum roll (7); said vacuum roll enables the removal of the excess amount of material applied by the perforating pins;
   c) driving the perforated material sheet (8) onto a cooling unit (9) thereby providing the fixing onto the walls of the holes of the perforations and the stabilization of the pattern of the so-obtained holes;
- a product selected from the group consisting of diapers, sanitary napkins, wound dressings, cosmetic product, personal care product and clothing, comprising the thermoplastic perforated film of the invention or obtainable from any one of the methods of the invention.

Further advantages and/or embodiments of the present invention will be evident from the following detailed description.

In each of the embodiment herein disclosed, the term "comprising" can be replaced by the term "consisting of".

### DETAILED DESCRIPTION OF THE FIGURES

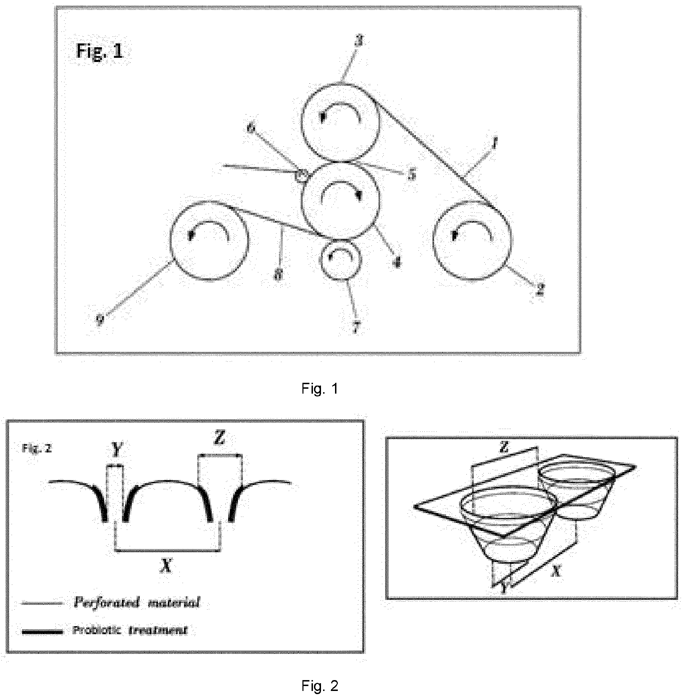

**Fig. 1****.** reports a schematic representation of the method for the production of the coated perforated materials of the invention comprising the following steps:
   a) Introducing a material sheet through a perforating nip (5) formed by a drum (4) carrying perforating pins and a female roll (3), while the perforating pins applied on the drum pass through a probiotic materials applicator (6) before becoming in contact with the material sheet.
   b) The perforated material sheet remains in contact with the pins of the drum roll up to a phase of separation from the pins due to the action of a vacuum roll (7); in addition said vacuum roll enables the removal of the excess amount of material applied by the perforating pins.
   c) The perforated material sheet (8) is then driven onto a cooling unit (9) which provides the fixing onto the walls of the holes of the perforations; said cooling unit also provides the stabilization of the pattern of the holes so obtained.
Fig. 2. reports an example of a hole and pattern of deposition of probiotic material.

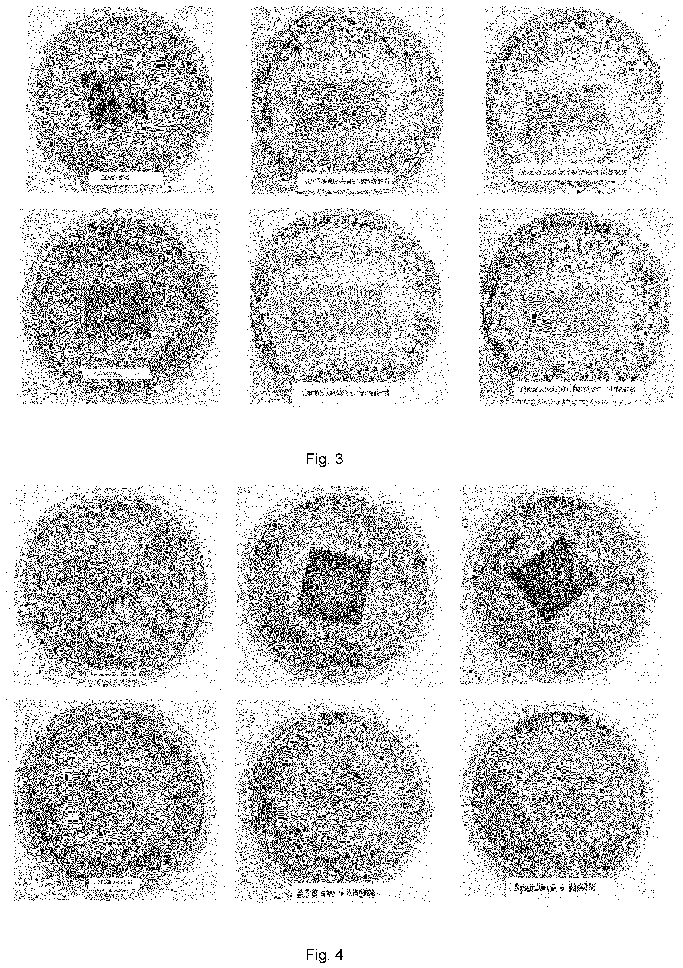

In Fig. 3 two non-woven fabrics: air-through-bonding (ATB) and spunlace were coated with two types of probiotic metabolites (Leuconostoc ferment filtrate and Lactobacillus ferment). The antimicrobial activity of the resultant coated fabrics was examined against Escherichia coli by the agar diffusion assay: pieces of coated fabrics were placed on agar surface previously inoculated with test microorganisms. After 24h incubation at 37-40 °C the capability of both coated fabrics in suppressing/inhibiting the growth of Escherichia coli was determined by the resulting inhibition growth areas.
In **Fig. 4** a PE perforated film, air-through-bonding (ATB) and spunlace non wovens were coated with a bacteriocin (nisin, trade name Delvonis) and retained activity against the indicator bacteria S. Aureus was assessed by the agar diffusion assay. The antimicrobial activity of the resultant extruded film was assessed. Pieces of materials not treated (control) and incorporating nisin were placed on agar surface previously inoculated with S. aureus. After 24h incubation at 37-40 °C the capability in suppressing/inhibiting the growth of S. aureus was determined by the resulting inhibition growth areas vs control.

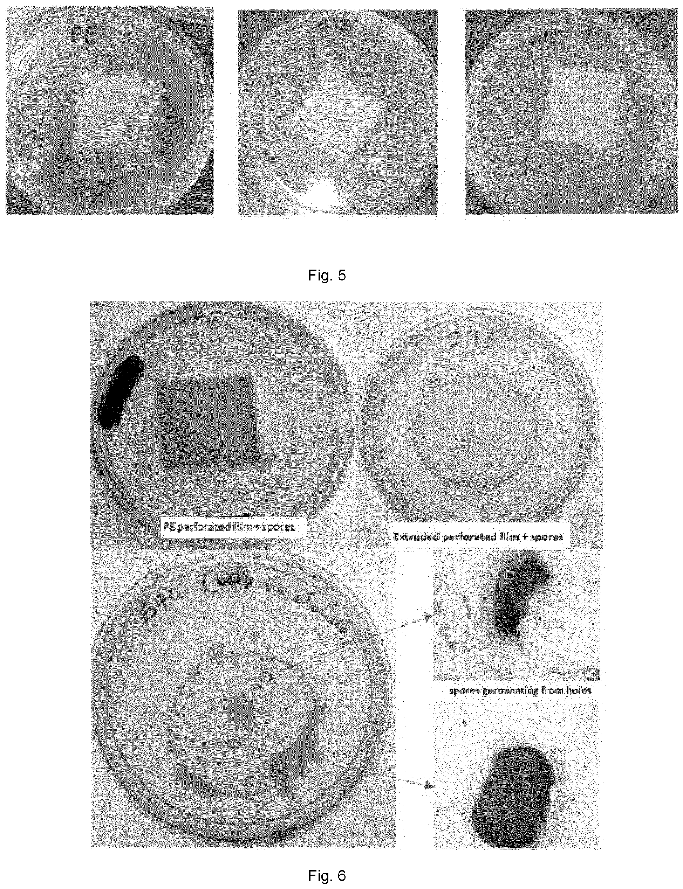

In **Fig. 5** viability of spores coated on a PE perforated film is determined by growth/germination biological assay which means that spores that survive the coating processing conditions perform colonies after inoculation and are detected as viable. Coated samples are cut into small pieces, then sterilized by soaking in in ethanol (96%) for 10 minutes. After sterilization, the samples are immersed in 10 ml of sterile physiological saline solution and then inoculated on nutrient agar plates and incubated at 37°C for 24 hours.
In **Fig. 6** viability of spores extruded within a PE perforated film is determined by growth/germination biological assay which means that spores that survive the extrution processing conditions perform colonies after inoculation and are detected as viable. Extruded samples are cut into small pieces, then sterilized by soaking in in ethanol (96%) for 10 minutes. After sterilization, the samples are immersed in 10 ml of sterile physiological saline solution and then inoculated on nutrient agar plates and incubated at 37°C for 24 hours.

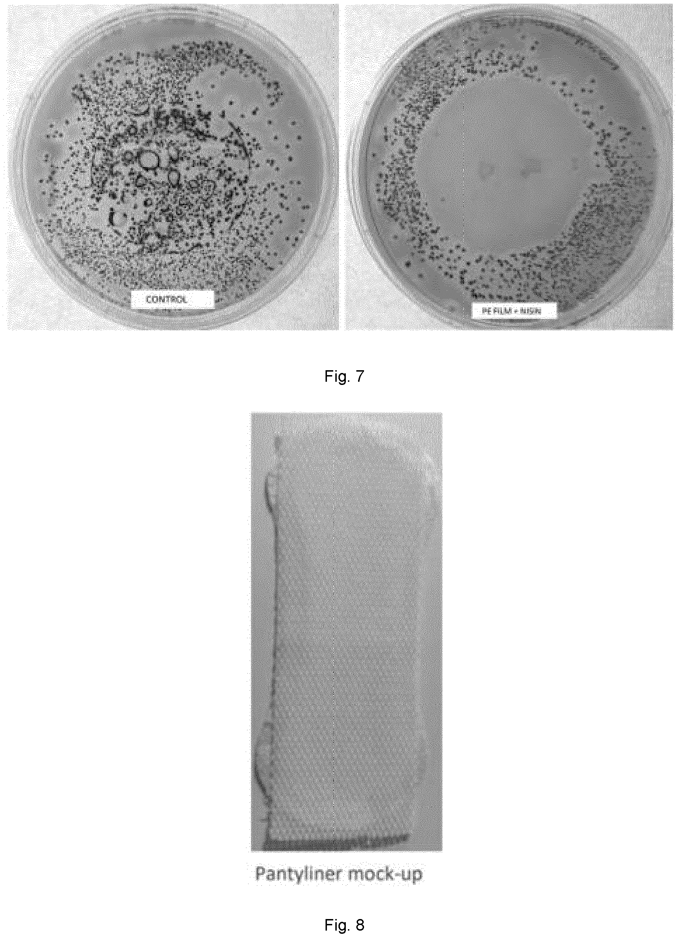

In **Fig. 7** a bacteriocin, nisin, was incorporated into a polyethylene-based plastic and retained activity against the indicator bacteria S. Aureus was assessed. 200 grams of powdered low density polyethylene was dry blended with 8gr of nisin in dried powder with salts and milk proteins under the trade name Delvonis. The active nisin content of the powder was 2,5% w/w. The nisin content in the film was therefore 0,1% by weight. Temperature of extrusion was 120°C, retention time in the extruder under was approximately 7 min. The antimicrobial activity of the resultant extruded film was assessed by the agar diffusion assay. Pieces of film were placed on agar surface previously inoculated with S. aureus. After 24h incubation at 37-40 °C the capability in suppressing/inhibiting the growth of S. aureus was determined by the resulting inhibition growth areas.

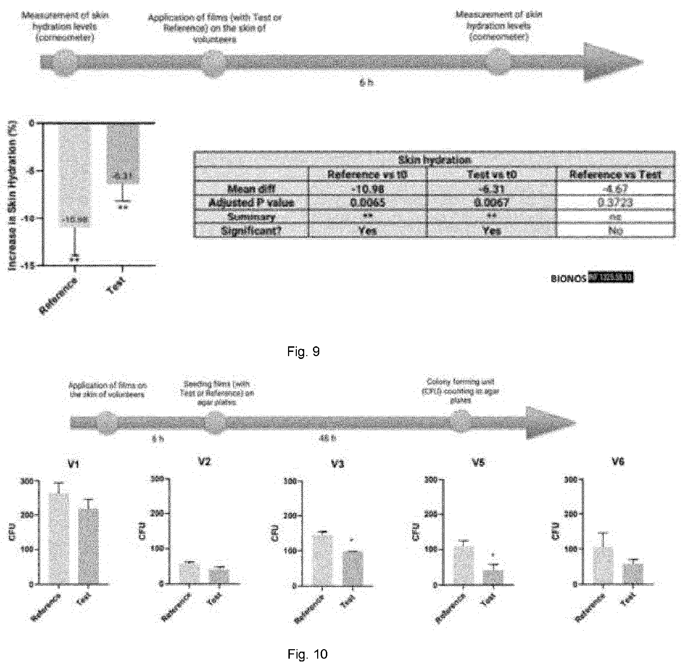

In **Fig. 9****,** representing a lactobacillus ferment forearm test, a double perforated PE film was produced and coated with Lactobacillus Ferment on industrial line. The impact of the postbiotic on the skin was evaluated in terms of hydration level after 6 hours of intimate contact with the forearm skin (vs reference) The results shows that the postbiotic coat tends to compensate (4,67%) the loss in hydration caused by reference.
In **Fig. 10****,** representing a lactobacillus ferment forearm test, the effectiveness of the postbiotic coating in inhibiting bacterial overgrowth (compared to placebo) was evaluated after contact of the treated substrate with the forearm skin (contact time: 6hrs). The results show that the treated sample tends to reduce bacterial proliferation caused by the occlusion.

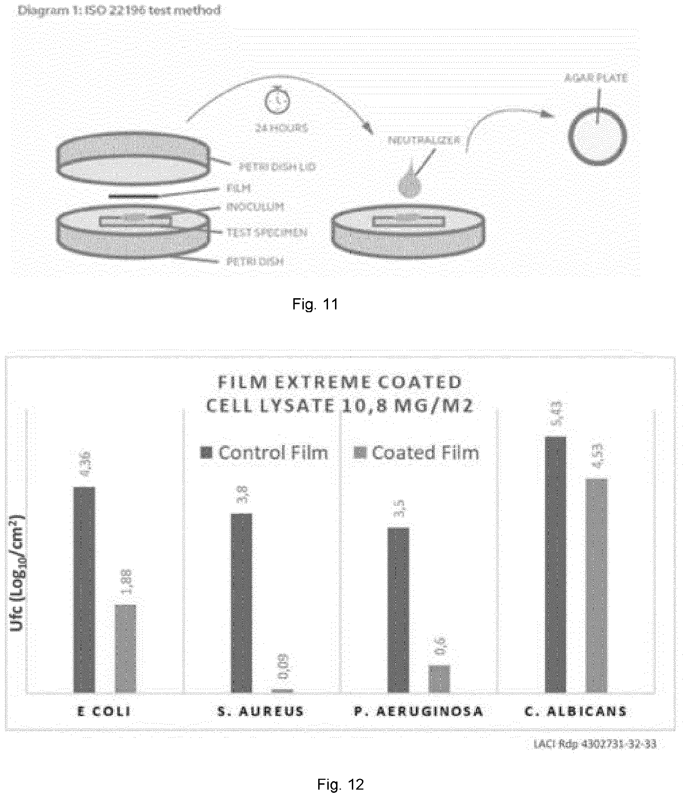

In **Fig. 11** it is depicted a scheme of the ISO22196 test metod procedure.
In **Fig. 12****,** Antibacterial activity vs pathogens was assessed on Double perforated PE film according the ISO22196 (Measurement of antibacterial activity on plastic and other non-porous surface). PE film coated with Lactobacillus Ferment is able to inhibit the growth of the tested bacteria E. Coli, S. Aureus, P. Aeruginosa and Candida Albicans.

### DETAILED DESCRIPTION OF THE INVENTION

The present invention relates to materials and process for their manufacturing, that can be incorporated in products thought for being in contact with human body such as absorbent hygienic products (baby diapers, adult incontinence diapers and women's personal hygiene products) comprising spores, soluble metabolites and/or cell lysates of probiotic micro-organism.

### Non-woven fabric material

The present invention relates to a non-woven fabric material comprising spores, soluble metabolites and/or cell lysates of a probiotic micro-organism.

In an embodiment, said probiotic micro-organism is incorporated within the fibres of said non-woven fabric. The term "incorporated within the fibres of said non-woven fabric material" means that the spores, soluble metabolites and/or cell lysates are added during the manufacturing process of the fibres of said non-woven fabric material and are able to remain viable during high-pressure and/or high-temperature conditions applied before and during the production process of the non-woven fabric material.

In another embodiment, said non-woven fabric material is coated with said probiotic micro-organism.

In an embodiment, said non-woven fabric material consists of perforated substrate of fibres. The perforated substrate permits the spores, metabolites and/or cell lysates of probiotic micro-organism, when the non-woven fabric material is in direct contact with the body and or body fluids, to directly interact with body/skin microbiota, thus being able to deliver their beneficial effects in preserving/restoring the microbial ecological balance. In an embodiment, the fibres are monocomponent, bicomponent or multicomponent fibres.

In a preferred embodiment, the fibres are polypropylene, polyethylene, polyester, PLA, viscose, lyocell, cotton, hemp, flax, cellulose, kapok fibres or a combination thereof.

In an embodiment, said fibres are combination of polyester and viscose, polyester and cotton, viscose and cotton, viscose and hemp, viscose and flax or PLA and viscose.

In an embodiment of the present invention, said probiotic micro-organism is one or more sporougenous bacteria of the genus Bacillus selected from Bacillus subtilis, Bacillus Licheniformis, Bacillus Polymyxa, Bacillus Circulans, Bacillus Coagulants, Bacillus Amyloliquefacens.

In a preferred embodiment, said probiotic micro-orgaism is at least one selected from *Bifidobacterium, Brevibacterium, Propionibacterium, Lactococcus, Streptococcus, Lactobacillus, Enterococcus, Pediococcus, Leuconostoc, Oenococcusa,* a lactic acid bacteria or a combination thereof.

In another embodiment, said probiotic micro-organism is at least one selected from *Lactobacillus acidophilus, Lactobacillus salivarius, Bifidobacterium lactis, Propionibacterium jensenii* or a combination thereof.

In a more preferred embodiment, said probiotic micro-organism is a probiotic mixture comprising *B*. *Subtilis* preferably from 20 to 30%, *B. Licheniformis* preferably from 10 to 20%, *B. Coagulants* preferably from 10 to 20%, *B. Polymyxa* preferably from 20 to 30%, *B. Amyloliquefacens* preferably from 10 to 20%.

In particular, in an embodiment of the invention, the probiotic micro-organism is present in an amount of from 10³ to 10⁹ CFU/g of material.

In another aspect, the present invention relates to a method of preparing a non-woven fabric material comprising into the fibres of said material spores, soluble metabolites or cell lysates of probiotic micro-organism, comprising the following steps:
i) mixing spores, soluble metabolites and/or cell lysates of probiotic micro-organism with pellets of a thermoplastic polymer in order to provide a mixture with a total probiotic concentration from 0,01% to 10% w/w;
ii) extruding said mixture obtained on point i) at a temperature from 100°C to 350°C, a pressure from 0,1 MPa to 40,0 MPa, average residence times from 1 to 10 min; or
   pelletizing, thermos-molding, compression molding, injection molding, thermo forming, impregnating, vacuum-forming, foaming or hot-melting said mixture obtained on point i) in order to obtain a thermoplastic fiber,
iii) producing a non-woven fabric material by a spunbond, meltblown, meltblown/spunbond combination, short fiber carding, or air-laid process using the thermoplastic fibres obtained from step ii).In addiction to spunbond and meltblow webs, can be made staple fibers (e.g. polypropylene fibers) to be used in non-woven carding process (e.g. fibres with length around 40 mm) or air-laid process (e.g. fibres with length around 2-6 mm).

In an embodiment, in said step i), the total probiotic concentration is from 0,1 to 10%, preferably in the range of 1,0 to 8%, more preferably in the range of 1,0 to 5,0% w/w.

In another embodiment, in said step ii) the temperature is from 120 to 300°C, preferably from 130 to 300°C, more preferably from 150 to 300°C, still more preferably the highest temperature is 220°C, 210°C, 200°C or even more preferably from 150 to 200°C.

In a further embodiment, in said step ii) the pressure is from 0,5 to 10,0 MPa, preferably from 0,5 to 5,0 MPa.

Moreover, in an embodiment, in said step ii) the average residence times are from 1 to 6 min, preferably from 1,5 to 4,5 min.

It is known by the skilled person that temperature, pressure and residence time used in the methods of the present invention may be adapted as much as possible to ensure maximal viability of the micro-organisms while still being able to manipulate the matrix material as desired.

In a preferred embodiment, said spores, metabolites and/or cell lysates of probiotic micro-organism are in the form of a powder or a lyophilized powder. Methods of preparing a powder or a lyophilized powder are well known to the skilled person.

In a further aspect, the present invention relates to a method of preparing a non-woven fabric material coated with spore, soluble metabolite and/or cell lysate of probiotic micro-organism, comprising the following steps:
a) mixing spores, soluble metabolites and/or cell lysates of probiotic micro-organism in order to provide a mixture with a total probiotic concentration from 0,01% to 10% w/w with respect to the weight of the non-woven fabric material, optionally with additives;
b) spraying or coating a non-woven fabric material with said mixture obtained in step a). The spraying or the coating of said non-woven fabric material can be subjected to modifications, if necessary, depending on the shape, thickness, tensile strength, breakage resistance, loading capacity and other structural and mechanical characteristics of the non-woven fabric material. The skilled person is able to determine the better conditions depending on the particular non-woven fabric material and on the apparatus used for its manufacturing.

In an embodiment, in said step i), the total probiotic concentration is from 0,1 to 10%, preferably in the range of 1,0 to 8%, more preferably in the range of 1,0 to 5,0% w/w.

In a preferred embodiment, when the probiotic micro-organism is mixed with additives, said additives are selected from ionic surfactants, cationic surfactants, amphoteric surfactants or mixtures thereof. Examples of said additives are Cirrasol PP682 marketed by Uniqema, Stantex S 6327 marketed by Cognis, Silastol PST marketed by Schill & Seilacher, Silwet L-7608 marketed by Momentive Performance Materials.

A further aspect of the present invention relates to a method for the preparation of a coated perforated non-woven fabric comprising the following steps:
a) introducing a non-woven fabric through a perforating nip (5) formed by a drum (4) carrying perforating pins and a female roll (3), while the perforating pins applied on the drum pass through a probiotic materials applicator (6) before becoming in contact with the non-woven fabric material;
b) keeping in contact the perforated non-woven fabric material with the pins of the drum roll up to a phase of separation from the pins due to the action of a vacuum roll (7); said vacuum roll enables the removal of the excess amount of material applied by the perforating pins;
c) driving the perforated material sheet (8) onto a cooling unit (9) which provides the fixing onto the walls of the holes of the perforationsand the stabilization of the pattern of the so-obtained holes.

In another aspect, the present invention relates to a product selected from the group consisting of diapers, sanitary napkins, wound dressings, cosmetic product, personal care product and clothing, comprising the non-woven fabric material of the present invention, or that is obtainable from any one of the methods of the present invention.

### Thermoplastic perforated film

The present invention also relates to a thermoplastic perforated film comprising spores, soluble metabolites and/or cell lysates of probiotic micro-organism.

In an embodiment, said probiotic micro-organism is incorporated into the polymer matrix of thermoplastic perforated film. The term "incorporated into the polymer matrix" means that the spores, soluble metabolites and/or cell lysates are added during the manufacturing process of the thermoplastic film and are able to remain viable during high-pressure and/or high-temperature conditions applied before and during the production process of the non-woven fabric material.

An example of thermoplastic plastic film is described in the ITCH 2006003 patent. In accordance with this patent, a polyperforated film of material (usually based on LDPE, LLDPE and HDPE) is extruded with cast technology. The film in a still plastic state is placed on a matrix where there are micro-openings with a density ranging from 140 holes per sq cm to 1024 holes per sq cm and immediately placed under vacuum, thus imploding the film and creating micro cones on it three dimensional.

The film is left in contact with the matrix for the time necessary to pass from the softening temperature to a temperature such as to allow the detachment of the film from the forming matrix. The film thus formed is ready for macro drilling.

A needle roller suitably thermoregulated at a temperature close to the thermoplastic film thermodrilling temperature is set in rotation and synchronized with a pair of perforated rollers with a density of holes equal to the density of the needles. Both perforated rolls can be temperature controlled. The perforated roller has the function of creating the three-dimensional cone and can be replaced by a brush roller with a high density of bristles. The perforated roller has the function of detaching the perforated film from the needles. The micro-perforated film is passed through the pair of rollers thus creating the three-dimensional macro hole; the film obtained is left in contact with the needles for the time necessary to obtain correct thermoforming. The polyperforated film can also be obtained with other methods known to the state of the art such as the use of blown extrusion and perforation using jets of compressed water. The perforated substrate permits the spores, metabolites and/or cell lysates of probiotic micro-organism, when the non-woven fabric material is in direct contact with the body and or body fluids, to directly interact with body/skin microbiota, thus being able to deliver their beneficial effects in preserving/restoring the microbial ecological balance.

In another embodiment, said thermoplastic perforated film is coated with said probiotic micro-organism.

In an embodiment of the present invention, said probiotic micro-organism is one or more sporougenous bacteria of the genus Bacillus selected from Bacillus subtilis, Bacillus Licheniformis, Bacillus Polymyxa, Bacillus Circulans, Bacillus Coagulants, Bacillus Amyloliquefacens.

In a preferred embodiment, said probiotic micro-orgaism is at least one selected from *Bifidobacterium, Brevibacterium, Propionibacterium, Lactococcus, Streptococcus, Lactobacillus, Enterococcus, Pediococcus, Leuconostoc, Oenococcusa,* a lactic acid bacteria or a combination thereof.

In another embodiment, said probiotic micro-organism is at least one selected from *Lactobacillus acidophilus, Lactobacillus salivarius, Bifidobacterium lactis, Propionibacterium jensenii* or a combination thereof.

In a more preferred embodiment, said probiotic micro-organism is a probiotic mixture comprising *B*. *Subtilis* preferably from 20 to 30%, *B. Licheniformis* preferably from 10 to 20%, *B. Coagulants* preferably from 10 to 20%, *B. Polymyxa* preferably from 20 to 30%, *B. Amyloliquefacens* preferably from 10 to 20%.

In particular, in an embodiment of the invention, the probiotic micro-organism is present in an amount of from 10³ to 10⁹ CFU/g of material.

In a preferred embodiment, said thermoplastic perforated film comprises polyethylene, polypropylene, polyester, PLA, viscose, lyocell, cotton, hemp, flax, cellulose, kapok fibres or a combination thereof, optionally further comprising additives.

In an embodiment, said fibres are combination of polyester and viscose, polyester and cotton, viscose and cotton, viscose and hemp, viscose and flax or PLA and viscose.

Examples of additives are stabilizers, surfactans and dyes, and are well known to the skilled person.

Reported herein are two formulations examples of the thermoplastic perforated film of the present invention:
Example 1: Extruded film
   - LDPE (low-density polyethylene) 44%
   - HDPE (high-density polyethylene) 54%
   - 1,45% CaCO₃ (stabilizer)
   - 0,05% non ionic surfactant
   - 0,5% spores of probiotic micro-organism
Example 2: Coated film
   - LDPE (low-density polyethylene) 44,5%
   - HDPE(low-density polyethylene) 54%
   - 1,4% TiO₂ (dye)
   - 0,05% non ionic surfactant
   - 0,1% spores of probiotic micro-organism

In a further aspect, the present invention relates to a method of preparing a thermoplastic film comprising spore, soluble metabolite and/or cell lysate of probiotic micro-organism into the polymer matrix comprising the following steps:
i) mixing spores, soluble metabolites and/or cell lysates of probiotic micro-organsim with pellets of a thermoplastic polymer in order to provide a mixture with a total probiotic concentration from 0,01% to 10% w/w;
ii) extruding said mixture obtained on point i) at a temperature from 100°C to 350°C, a pressure from 0,1 MPa to 40,0 MPa, average residence times from 1 to 10 min; or
pelletizing, thermos-molding, compression molding, injection molding, thermo forming, impregnating, vacuum-forming, foaming or hot-melting said mixture obtained on point i) in order to obtain a thermoplastic film,

In an embodiment, in said step i), the total probiotic concentration is from 0,1 to 10%, preferably in the range of 1,0 to 8%, more preferably in the range of 1,0 to 5,0% w/w.

In another embodiment, in said step ii) the temperature is from 120 to 300°C, preferably from 130 to 300°C, more preferably from 150 to 300°C, still more preferably the highest temperature is 220°C, 210°C, 200°C or even more preferably from 150 to 200°C.

In a further embodiment, in said step ii) the pressure is from 0,5 to 10,0 MPa, preferably from 0,5 to 5,0 MPa.

Moreover, in an embodiment, in said step ii) the average residence times are from 1 to 6 min, preferably from 1,5 to 4,5 min.

It is known by the skilled person that temperature, pressure and residence time used in the methods of the present invention may be adapted as much as possible to ensure maximal viability of the micro-organisms while still being able to manipulate the matrix material as desired.

In a preferred embodiment, said spores, metabolites and/or cell lysates of probiotic micro-organism are in the form of a powder or a lyophilized powder. Methods of preparing a powder or a lyophilized powder are well known to the skilled person.

Furthermore, in an embodiment, the method further comprises a step of perforating said thermoplastic film obtained in step ii) according to the methods known by the skilled person.

In a further aspect, the present invention relates to a method of preparing a thermoplastic film coated with spore, soluble metabolite and/or cell lysate of probiotic micro-organism, comprising the following steps:
a) mixing spores, soluble metabolites and/or cell lysates of probiotic micro-organism in order to provide a mixture with a total probiotic concentration from 0,01% to 10% w/w with respect to the weight of the thermoplastic film, optionally also with additives;
b) spraying or coating a thermoplastic film with said mixture obtained in step a).

The spraying or the coating of said thermoplastic perforated film can be subjected to modifications, if necessary, depending on the shape, thickness, tensile strength, breakage resistance, loading capacity and other structural and mechanical characteristics of the thermoplastic perforated film. The skilled person is able to determine the better conditions depending on the particular thermoplastic perforated film and on the apparatus used for its manufacturing.

In an embodiment, in said step i), the total probiotic concentration is from 0,1 to 10%, preferably in the range of 1,0 to 8%, more preferably in the range of 1,0 to 5,0% w/w.

In a preferred embodiment, when the probiotic micro-organism is mixed with additives, said additives are selected from ionic surfactants, cationic surfactants, amphoteric surfactants or mixtures thereof. Examples of said additives are Cirrasol PP682 marketed by Uniqema, Stantex S 6327 marketed by Cognis, Silastol PST marketed by Schill & Seilacher, Silwet L-7608 marketed by Momentive Performance Materials.

In another aspect, the present invention relates to a method for the preparation of a perforated thermoplastic film coated with spores, soluble metabolites and/or cell lysates of probiotic micro-organism, comprising the following steps:
a) introducing a thermoplastic film sheet through a perforating nip (5) formed by a drum (4) carrying perforating pins and a female roll (3), while the perforating pins applied on the drum pass through a probiotic materials applicator (6) before becoming in contact with the thermoplastic film sheet;
b) keeping in contact the perforated thermoplastic film sheet with the pins of the drum roll up to a phase of separation from the pins due to the action of a vacuum roll (7); said vacuum roll enables the removal of the excess amount of material applied by the perforating pins;
c) driving the perforated material sheet (8) onto a cooling unit (9) thereby providing the fixing onto the walls of the holes of the perforations and the stabilization of the pattern of the so-obtained holes.

In a final aspect, the present invention relates to a product selected from the group consisting of diapers, sanitary napkins, wound dressings, cosmetic product, personal care product and clothing, comprising the non-woven fabric material of the present invention, or that is obtainable from any one of the methods of the present invention.

### EXAMPLES

### Example 1. Antimicrobial activity of coated materials

Two non-woven fabrics: air-through-bonding (ATB) and spunlace were coated with two types of probiotic metabolites (Leuconostoc ferment filtrate and Lactobacillus ferment)
The antimicrobial activity of the resultant coated fabrics was examined against Escherichia coli by the agar diffusion assay: pieces of coated fabrics were placed on agar surface previously inoculated with test microorganisms.

After 24h incubation at 37-40 °C the capability of both coated fabrics in suppressing/inhibiting the growth of Escherichia coli was determined by the resulting inhibition growth areas.

### Example 2.

A PE perforated film, air-through-bonding (ATB) and spunlace non wovens were coated with a bacteriocin (nisin, trade name Delvonis) and retained activity against the indicator bacteria S. Aureus was assessed by the agar diffusion assay.

The antimicrobial activity of the resultant extruded film was assessed.

Pieces of materials not treated (control) and incorporating nisin were placed on agar surface previously inoculated with S. aureus.

After 24h incubation at 37-40 °C the capability in suppressing/inhibiting the growth of S. aureus was determined by the resulting inhibition growth areas vs control.

### Example 3. Viability of spores coated on PE perforated film

Viability of spores coated on a PE perforated film is determined by growth/germination biological assay which means that spores that survive the coating processing conditions perform colonies after inoculation and are detected as viable.

Coated samples are cut into small pieces, then sterilized by soaking in in ethanol (96%) for 10 minutes.

After sterilization, the samples are immersed in 10 ml of sterile physiological saline solution and then inoculated on nutrient agar plates and incubated at 37°C for 24 hours.

### Example 4. Resistance of extruded spores

Viability of spores extruded within a PE perforated film is determined by growth/germination biological assay which means that spores that survive the extrusion processing conditions perform colonies after inoculation and are detected as viable.

Coated samples are cut into small pieces, then sterilized by soaking in in ethanol (96%) for 10 minutes.

After sterilization, the samples are immersed in 10 ml of sterile physiological saline solution and then inoculated on nutrient agar plates and incubated at 37°C for 24 hours.

### Example 5. Antimicrobial activity of bacteriocin incorporating extruded film

A bacteriocin, nisin, was incorporated into a polyethylene based plastic and retained activity against the indicator bacteria S. Aureus was assessed.

### Plastic film preparation

200 grams of powdered low density polyethylene was dry blended with 8gr of nisin in dried powder with salts and milk proteins under the trade name Delvonis. The active nisin content of the powder was 2,5% w/w. The nisin content in the film was therefore 0,1% by weight.

Temperature of extrusion was 120°C, retention time in the extruder under was approximately 7 min.

The antimicrobial activity of the resultant extruded film was assessed by the agar diffusion assay.

Pieces of film were placed on agar surface previously inoculated with S. aureus.

After 24h incubation at 37-40 °C the capability in suppressing/inhibiting the growth of S. aureus was determined by the resulting inhibition growth areas.

Data show that either spores and metabolites retain their properties (ability of spores of germinating and antimicrobial activity of the metabolites and bacteriocins) when coated end/or extruded in the materials, thus "activating" the materials in which are contained/deposited.

In turn these properties and final benefits will be delivered through the finished product on the body during the product usage.

### Example 6

### SOLUBLE METABOLITES AND/OR CELL LYSATES (POSTBIOTICS)

Experimental data have been produced to support the beneficial effects for humans of materials coated with soluble metabolites and/or cell lysates of probiotic (postbiotic) microorganisms.

### In vivo tests

The objective of the study was to evaluate the effects on the hydration state of the skin and on the microbial load after prolonged contact with the material treated with the postbiotic substance.

In the clinical study, 5 volunteers were subjected to 6-hour contact with a film on which the probiotic substance was deposited (Test) and the same untreated film (reference). The films were applied to the external surface of a panty liner and then placed on the skin of the volunteers' forearm.

To simulate conditions of use, the products were kept in close contact with the skin using an elastic net for a period of 6 hours (Fig. 9).

### EFFECT ON SKIN HYDRATION

The degree of hydration of the skin was evaluated, using the Corneometro^{®} CM 825, before (T0) and after 6 hours (T6) (Fig. 9).

After 6 hours (T6) the skin in contact with the untreated film (reference) appears to have lost a level of hydration equal to 11% compared to the value at T0.

The skin that was in contact with the film treated with the post biotic instead reduced its initial hydration level by 6.31%.

Therefore the coating with the post biotic material tends to compensate (4.67%) the loss of hydration caused by the occlusion due to the long-lasting intimate contact with the plastic film.

### EFFECT ON SKIN MICROBIAL FLORA

To evaluate the effect on the microbial flora, the films, after 6 hours of contact with the skin, were aseptically placed on TSA agar, incubated for 1 hour and then removed from the culture medium.

The plates were then incubated for a period of 48 hours at 37°C and colony forming units (CFU) were counted visually.

The material treated (test) with the postbiotic after contact with the skin has a lower microbial flora than the untreated material (reference), which indicates that the presence of the postbiotic material reduces the bacterial proliferation caused by the occlusion (Fig. 10).

### In vitro tests

To confirm the antimicrobial properties of the material conferred by the treatment with the post biotic, the in vitro test was carried out according to the ISO 22196:2011 standard which is a specific method for evaluating the antibacterial activity of plastic films.

The method consists of inoculating the surface of a film sample (sized 50 mm × 50 mm and often no more than 10 mm) with 400 µl of solution containing the microorganism under study.

The inoculated sample is then covered with 40 mm x 40 mm film and placed in a Petri dish on culture medium.

This procedure is applied to untreated material (control) and to material whose surface has been treated with the postbiotic (test).

Petri dishes containing the inoculated samples are incubated at a temperature of 35°C and a relative humidity of no less than 90% for 24 hours.

Next, 10 ml of neutralizer is added to the Petri dish. A 10-fold serial dilution is performed on the neutralizer and 10 ml of each dilution is plated on plate count agar. The plates are incubated at 35°C for 40-48 hours.

The log reduction is determined by comparing the bacterial counts of the treated test samples and the untreated control.

The graph in Fig. 12 shows that on the surface of the film treated with postbiotic the growth of the microorganisms under study is significantly reduced.

### Example 7

### PROBIOTICS SPORES

The production process of the polyperforated plastic film containing spores of probiotic microorganisms has been defined and the viability of the spore-forming microorganisms at high temperatures and pressures has been confirmed.

LDPE pellets containing 5% CaCO₃ powder carrying a quantity equal to 20.9x10⁸ CFU/g of Bacillus Subtilis spores were prepared.

Polyperforated films were then made by mixing 5% and 10% spore pellets with untreated LDPE.

The extrusion of the resulting mixture was carried out according to the diagram shown and by setting a temperature profile where, in the polymer melting zone, the temperature was 210°C, and then progressively decreased to 170°C at the exit. The total residence time during extrusion is 2-3 minutes. The permanence at the temperature of 210°C can be estimated at 60-90 seconds.

The resistance of the spores extruded in the pellets and therefore after the creation of a film from the same pellets was determined by biological growth/germination test on TSA agar culture medium and kept in an incubator at 37°C for 2 days.

The spores were confirmed to survive the conditions of the process of pelleting, subsequent extrusion and then puncturing to form the relevant colonies.

## Claims

1. A thermoplastic perforated film comprising spores, soluble metabolites and/or cell lysates of probiotic micro-organism for use as a solid support to be used as top layer in a finished product for personal hygiene.

2. The thermoplastic perforated film according to claim 1, wherein said spores, soluble metabolites and/or cell lysates of probiotic micro-organism are incorporated into the polymer matrix of said thermoplastic perforated film.

3. The thermoplastic perforated film according to claim 1, wherein said thermoplastic perforated film is coated with said spores, soluble metabolites and/or cell lysates of probiotic micro-organisms.

4. The thermoplastic perforated film according to any one of claims 1 to 3, wherein said spores, soluble metabolites and/or cell lysates of probiotic micro-organisms are one or more sporougenous bacteria of the genus Bacillus selected from Bacillus subtilis, Bacillus Licheniformis, Bacillus Polymyxa, Bacillus Circulans, Bacillus Coagulants, Bacillus Amyloliquefacens, preferably is at least one selected from *Bifidobacterium, Brevibacterium, Propionibacterium, Lactococcus, Streptococcus, Lactobacillus, Enterococcus, Pediococcus, Leuconostoc, Oenococcusa,* a lactic acid bacteria or a combination thereof, still more preferably is at least one selected from *Lactobacillus acidophilus, Lactobacillus salivarius, Bifidobacterium lactis, Propionibacterium jensenii* or a combination thereof.

5. The thermoplastic perforated film according to anyone of claims 1 to 4, wherein the at least one spore, soluble metabolite and/or cell lysate of probiotic micro-organism is a probiotic mixture comprising *B. Subtilis* preferably from 20 to 30%, *B. Licheniformis* preferably from 10 to 20%, *B*. *Coagulants* preferably from 10 to 20%, *B. Polymyxa* preferably from 20 to 30%, *B*. *Amyloliquefacens* preferably from 10 to 20%.

6. The thermoplastic perforated film according to anyone claims 1 to 5, wherein the spores, soluble metabolites and/or cell lysates of probiotic micro-organisms are present in an amount of from 10³ to 10⁹ CFU/g of material.

7. The thermoplastic perforated film according to any one of claims 1 to 6, wherein said thermoplastic perforated film comprises polyethylene, polypropylene, polyester, PLA, viscose, lyocell, cotton, hemp, flax, cellulose, kapok fibres, or a combination thereof, optionally further comprising additives.

8. A method of preparing a thermoplastic film comprising spore, soluble metabolite and/or cell lysate of probiotic micro-organism according to any one of claims 1, 2 and 4-7, preferably in the form of a powder or a lyophilized powder, into the polymer matrix comprising the following steps:
i) mixing spores, soluble metabolites and/or cell lysates of probiotic micro-organism with pellets of a thermoplastic polymer, preferably LDPE, in order to provide a mixture with a total probiotic concentration from 0,01% to 10% w/w, preferably 5% or 10% w/w;
ii) extruding said mixture obtained on point i) at a temperature from 100°C to 350°C, preferably 210°C, a pressure from 0,1 MPa to 40,0 MPa, average residence times from 1 to 10 min, preferably from 2 to 3 min; or
pelletizing, thermos-molding, compression molding, injection molding, thermoforming, impregnating, vacuum-forming, foaming or hot-melting said mixture obtained on point i)
in order to obtain a thermoplastic film.

9. The method according to claim 8, further comprising a step of perforating said thermoplastic film obtained in step ii).

10. The method according to claim 8 or 9, wherein said thermoplastic polymer is LDPE, and comprises from 1 to 10% w/w of CaCO₃, preferably 5% w/w.

11. The method according to anyone of claims 8 to 10, wherein said spores, soluble metabolites and/or cell lysates of probiotic micro-organism mixed with pellets of a thermoplastic polymer in step i) are in quantity from 1x10⁶ to 1x10¹⁰ CFU/g, preferably 20.9x10⁸ CFU/g.

12. The method according to anyone of claims 8 to 11, wherein said spores are Bacillus Subtilis spores.

13. A method of preparing a thermoplastic film coated with spore, soluble metabolite and/or cell lysate of probiotic micro-organism according to any one of claims 1 and 3-7, comprising the following steps:
a) mixing spores, soluble metabolites and/or cell lysates of probiotic micro-organism in order to provide a mixture with a total probiotic concentration from 0,01% to 10% w/w with respect to the weight of the thermoplastic perforated film, optionally also with additives;
b) spraying or coating a thermoplastic film with said mixture obtained in step a).

14. A product selected from the group consisting of diapers, sanitary napkins, wound dressings, cosmetic product, personal care product and clothing, comprising the thermoplastic perforated film of any one of claims 1 to 7 or obtainable from any one of the methods according to claims 8 to 13.

## Patentansprüche

1. Thermoplastische perforierte Folie, umfassend Sporen, lösliche Metaboliten und/oder Zelllysate eines probiotischen Mikroorganismus zur Verwendung als ein fester Träger, der als Deckschicht in einem Fertigprodukt für Körperhygiene zu verwenden ist.

2. Thermoplastische perforierte Folie nach Anspruch 1, wobei die Sporen, die löslichen Metaboliten und/oder die Zelllysate des probiotischen Mikroorganismus in die Polymermatrix der thermoplastischen perforierten Folie eingearbeitet sind.

3. Thermoplastische perforierte Folie nach Anspruch 1, wobei die thermoplastische perforierte Folie mit den Sporen, den löslichen Metaboliten und/oder den Zelllysaten von probiotischen Mikroorganismen beschichtet ist.

4. Thermoplastische perforierte Folie nach einem der Ansprüche 1 bis 3, wobei die Sporen, die löslichen Metaboliten und/oder die Zelllysate der probiotischen Mikroorganismen ein oder mehrere sporenbildende Bakterien der Gattung Bacillus sind, die aus Bacillus subtilis, Bacillus Licheniformis, Bacillus Polymyxa, Bacillus Circulans, Bacillus Coagulants, Bacillus Amyloliquefacens ausgewählt sind, vorzugsweise mindestens eines ist, das aus *Bifidobacterium, Brevibacterium, Propionibacterium, Lactococcus, Streptococcus, Lactobacillus, Enterococcus, Pediococcus, Leuconostoc, Oenococcusa,* einem Milchsäurebakterium oder einer Kombination davon ausgewählt ist, noch mehr bevorzugt mindestens eines ist, das aus *Lactobacillus acidophilus, Lactobacillus salivarius, Bifidobacterium lactis, Propionibacterium jensenii* oder einer Kombination davon ausgewählt ist.

5. Thermoplastische perforierte Folie nach einem der Ansprüche 1 bis 4, wobei die mindestens eine Spore, der lösliche Metabolit und/oder das Zelllysat des probiotischen Mikroorganismus eine probiotische Mischung, umfassend *B. Subtilis* vorzugsweise von 20 bis 30 %, *B. Licheniformis* vorzugsweise von 10 bis 20 %, *B. Coagulants* vorzugsweise von 10 bis 20 %, *B. Polymyxa* vorzugsweise von 20 bis 30 %, *B. Amyloliquefacens* vorzugsweise von 10 bis 20 %, ist.

6. Thermoplastische perforierte Folie nach einem der Ansprüche 1 bis 5, wobei die Sporen, löslichen Metaboliten und/oder Zelllysate der probiotischen Mikroorganismen in einer Menge von 10³ bis 10⁹ KbE/g Material vorhanden sind.

7. Thermoplastische perforierte Folie nach einem der Ansprüche 1 bis 6, wobei die thermoplastische perforierte Folie Polyethylen, Polypropylen, Polyester, PLA, Viskose, Lyocell, Baumwolle, Hanf, Flachs, Cellulose, Kapokfasern oder eine Kombination davon, gegebenenfalls ferner umfassend Zusatzstoffe, umfasst.

8. Verfahren zum Anfertigen einer thermoplastischen Folie, umfassend eine Spore, einen löslichen Metaboliten und/oder ein Zelllysat des probiotischen Mikroorganismus nach einem der Ansprüche 1, 2 und 4 bis 7, vorzugsweise in Form eines Pulvers oder eines lyophilisierten Pulvers, hinein in die Polymermatrix, umfassend die folgenden Schritte:
i) Mischen von Sporen, löslichen Metaboliten und/oder Zelllysaten des probiotischen Mikroorganismus mit Pellets eines thermoplastischen Polymers, vorzugsweise LDPE, um eine Mischung mit einer gesamten probiotischen Konzentration von 0,01 Gew.-% bis 10 Gew.-%, vorzugsweise 5 Gew.-% oder 10 Gew.-%, bereitzustellen;
ii) Extrudieren der in Punkt i) erhaltenen Mischung bei einer Temperatur von 100 °C bis 350 °C, vorzugsweise 210 °C, einem Druck von 0,1 MPa bis 40,0 MPa, durchschnittlichen Verweilzeiten von 1 bis 10 min, vorzugsweise von 2 bis 3 min; oder
Pelletieren, Thermo-Pressen, Formpressen, Spritzgießen, Thermoformen, Imprägnieren, Vakuumformen, Aufschäumen oder Heißschmelzen der in Punkt i) erhaltenen Mischung, um eine thermoplastische Folie zu erhalten.

9. Verfahren nach Anspruch 8, ferner umfassend einen Schritt eines Perforierens der in Schritt ii) erhaltenen thermoplastischen Folie.

10. Verfahren nach Anspruch 8 oder 9, wobei das thermoplastische Polymer LDPE ist und zu 1 bis 10 Gew.-%, vorzugsweise zu 5 Gew.-%, CaCO₃ umfasst.

11. Verfahren nach einem der Ansprüche 8 bis 10, wobei die Sporen, die löslichen Metaboliten und/oder die Zelllysate des probiotischen Mikroorganismus, die in Schritt i) mit Pellets eines thermoplastischen Polymers gemischt werden, in einer Anzahl von 1x10⁶ bis 1x10¹⁰ KbE/g, vorzugsweise 20,9x10⁸ KbE/g, vorliegen.

12. Verfahren nach einem der Ansprüche 8 bis 11, wobei die Sporen Bacillus-Subtilis-Sporen sind.

13. Verfahren zum Anfertigen einer thermoplastischen Folie, die mit einer Spore, einem löslichen Metabolit und/oder einem Zelllysat des probiotischen Mikroorganismus beschichtet ist, nach einem der Ansprüche 1 und 3 bis 7, umfassend die folgenden Schritte:
a) Mischen von Sporen, löslichen Metaboliten und/oder Zelllysaten des probiotischen Mikroorganismus, um eine Mischung mit einer gesamten probiotischen Konzentration von 0,01 Gew.-% bis 10 Gew.-%, bezogen auf das Gewicht der thermoplastischen perforierten Folie, gegebenenfalls auch mit Zusatzstoffen, bereitzustellen;
b) Besprühen oder Beschichten einer thermoplastischen Folie mit der in Schritt a) erhaltenen Mischung.

14. Produkt, das aus der Gruppe ausgewählt ist, bestehend aus Windeln, Sanitärbinden, Wundverbänden, einem kosmetischen Produkt, einem Körperpflegeprodukt und Kleidung, umfassend die thermoplastische perforierte Folie nach einem der Ansprüche 1 bis 7 oder erhaltbar aus einem beliebigen der Verfahren nach den Ansprüchen 8 bis 13.

## Revendications

1. Film perforé thermoplastique comprenant des spores, des métabolites solubles et/ou des lysats cellulaires de micro-organisme probiotique pour utilisation en tant que support solide à utiliser en guise de couche supérieure dans un produit fini pour l'hygiène personnelle.

2. Film perforé thermoplastique selon la revendication 1, dans lequel lesdits spores, métabolites solubles et/ou lysats cellulaires de micro-organisme probiotique sont incorporés dans la matrice de polymère dudit film perforé thermoplastique.

3. Film perforé thermoplastique selon la revendication 1, dans lequel ledit film perforé thermoplastique est revêtu desdites spores, métabolites solubles et/ou lysats cellulaires de micro-organismes probiotiques.

4. Film perforé thermoplastique selon l'une quelconque des revendications 1 à 3, dans lequel lesdits spores, métabolites solubles et/ou lysats cellulaires de micro-organismes probiotiques sont une ou plusieurs bactéries sporougeuses du genre Bacillus choisies parmi des Bacillus subtilis, Bacillus licheniformis, Bacillus polymyxa, Bacillus circulans, Bacillus coagulants, Bacillus amyloliquefacens, est de préférence au moins l'un choisi parmi des *Bifidobacterium, Brevibacterium, Propionibacterium, Lactococcus, Streptococcus, Lactobacillus, Enterococcus, Pediococcus, Leuconostoc, Oenococcusa,* une bactérie lactique ou une combinaison de ceux-ci, encore plus préférablement est au moins l'un choisi parmi des *Lactobacillus acidophilus, Lactobacillus salivarius, Bifidobacterium lactis, Propionibacterium jensenii* ou une combinaison de ceux-ci.

5. Film perforé thermoplastique selon l'une quelconque des revendications 1 à 4, dans lequel l'au moins une spore, métabolite soluble et/ou lysat cellulaire de micro-organisme probiotique est un mélange probiotique comprenant *B. Subtilis* de préférence de 20 à 30 %, *B. licheniformis* de préférence de 10 à 20 %, *B. Coagulants* de préférence de 10 à 20 %, *B. Polymyxa* de préférence de 20 à 30 %, *B. Amyloliquefacens* de préférence de 10 à 20 %.

6. Film perforé thermoplastique selon l'une quelconque des revendications 1 à 5, dans lequel les spores, métabolites solubles et/ou lysats cellulaires de micro-organismes probiotiques sont présents en une quantité allant de 10³ à 10⁹ UFC/g de matériau.

7. Film perforé thermoplastique selon l'une quelconque des revendications 1 à 6, dans lequel ledit film perforé thermoplastique comprend du polyéthylène, polypropylène, polyester, PLA, viscose, lyocell, coton, chanvre, lin, cellulose, fibres de kapok, ou une combinaison de ceux-ci, comprenant éventuellement en outre des additifs.

8. Procédé de préparation d'un film thermoplastique comprenant une spore, un métabolite soluble et/ou un lysat cellulaire de micro-organisme probiotique selon l'une quelconque des revendications 1, 2 et 4 à 7, de préférence sous forme de poudre ou de poudre lyophilisée, dans la matrice de polymère comprenant les étapes suivantes :
i) le mélange des spores, des métabolites solubles et/ou des lysats cellulaires de micro-organisme probiotique avec des pastilles d'un polymère thermoplastique, de préférence du LDPE, afin d'obtenir un mélange avec une concentration totale en probiotique de 0,01 % à 10 % p/p, de préférence 5 % ou 10 % p/p ;
ii) l'extrusion dudit mélange obtenu au point i) à une température allant de 100 °C à 350 °C, de préférence 210 °C, une pression allant de 0,1 MPa à 40,0 MPa, des temps de séjour moyens allant de 1 à 10 min, de préférence de 2 à 3 min ; ou
la granulation, thermomoulage, moulage par compression, moulage par injection, thermoformage, imprégnation, formage sous vide, moussage ou fusion à chaud dudit mélange obtenu au point i) afin d'obtenir un film thermoplastique.

9. Procédé selon la revendication 8, comprenant en outre une étape de perforation dudit film thermoplastique obtenu à l'étape ii).

10. Procédé selon la revendication 8 ou 9, dans lequel ledit polymère thermoplastique est du LDPE, et comprend de 1 à 10 % p/p de CaCO₃, de préférence 5 % p/p.

11. Procédé selon l'une quelconque des revendications 8 à 10, dans lequel lesdites spores, métabolites solubles et/ou lysats cellulaires de micro-organisme probiotique mélangés à des granulés d'un polymère thermoplastique à l'étape i) sont en quantité de 1x10⁶ à 1x10¹⁰ UFC/g, de préférence 20,9x10⁸ UFC/g.

12. Procédé selon l'une quelconque des revendications 8 à 11, dans lequel lesdites spores sont des spores de Bacillus Subtilis.

13. Procédé de préparation d'un film thermoplastique revêtu de spores, de métabolite soluble et/ou de lysat cellulaire de micro-organisme probiotique selon l'une quelconque des revendications 1 et 3 à 7, comprenant les étapes suivantes :
a) le mélange des spores, des métabolites solubles et/ou des lysats cellulaires de micro-organisme probiotique afin d'obtenir un mélange avec une concentration totale en probiotique de 0,01 % à 10 % p/p par rapport au poids du film perforé thermoplastique, éventuellement également avec des additifs ;
b) la pulvérisation ou le revêtement d'un film thermoplastique avec ledit mélange obtenu à l'étape a).

14. Produit choisi dans le groupe constitué de couches, serviettes hygiéniques, pansements, produits cosmétiques, produits de soins personnels et vêtements, comprenant le film perforé thermoplastique selon l'une quelconque des revendications 1 à 7 ou pouvant être obtenu par l'un quelconque des procédés selon les revendications 8 à 13.
